# EUROPÄISCHE PATENTSCHRIFT

(11) **EP 0 369 936 B1**
(45) Veröffentlichungstag und Bekanntmachung des Hinweises auf die Patenterteilung: **13.04.1994**
(21) Anmeldenummer: 89730213.9
(22) Anmeldetag: 19.11.1989
(51) Int. Cl.: A61B 1/00

(54) **Endoskop**
Endoscope
Endoscope

(30) Priorität: 18.11.1988 DE 8814573 U
(43) Veröffentlichungstag der Anmeldung: 23.05.1990
(73) Patentinhaber: Effner Biomet GmbH, D-12247 Berlin (DE)
(72) Erfinder: Anapliotis, Emmanuel, D-1000 Berlin 33 (DE); Schich, Gisbert, D-8800 Ansbach (DE)
(74) Vertreter: Christiansen, Henning, Dipl.-Ing.

(56) Entgegenhaltungen:
- DE-A- 3 727 190
- DE-B- 1 117 256
- GB-A- 1 311 018
- US-A- 3 297 022
- US-A- 4 369 768

## Beschreibung

Die Erfindung betrifft ein Endoskop der im Oberbegriff des Anspruchs 1 angegebenen Art.

Derartige Endoskope dienen insbesondere in Form von Athroskopen der Untersuchung und Behandlung akuter Gelenkinnenräume, meist des Kniegelenks. Vorzugsweise zur Abklärung von Meniskusverletzungen ist die Arthroskopie ein häufig angewandtes Verfahren.

Sehr oft werden Endoskope in Kombination mit chirurgischen Instrumenten nicht nur zur Beobachtung, sondern auch zur Beleuchtung des Operationsgebietes eingesetzt. Endoskope müssen sich daher sowohl durch hervorragende optische Parameter, als auch durch exzellente Kaltlichtbeleuchtung hoher Intensität auszeichnen.

Nachteilig bei bekannten Endoskopen ist die Tatsache, daß wegen der gedrängten Bauweise die Querschnitte von Optik und Lichtleitern nur relativ gering gewählt werden könne, insbesondere, wenn mit dem Endoskopschaft noch Spülkanäle oder dergleichen zusammengefaßt werden.

Weiterhin ist bei bekannten Endoskopen nachteilig, daß Beobachtungs- und Beleuchtungsbaugruppe fest miteinander verbunden sind. Im Falle einer - unter Operationsbedingungen relativ häufig auftretenden - Beschädigung der Optik des Endoskops müssen damit die vollständige Einheit ersetzt oder einer Reparaturstelle zugeführt werden. Die Reparatur gestaltet sich wegen der notwendigen komplizierten Demontage stets relativ aufwendig.

Da wegen des Entwicklungstrends zu immer leistungsstärkeren, komplizierteren und teureren Geräten mit immer enger werdenden speziellen Einsatzgebieten die Reparaturen nur in speziellen dafür eingerichteten Werkstätten ausgeführt werden können, steigen die mit einer Reparatur verbundenen Kosten ständig. vielfach können die umfangreichen und aufwendigen Wartungsarbeiten nur noch von den Servicespezialisten der Herstellerfirmen durchgeführt werden.

Ein weiteres aus der US-A-3 297 022 bekanntes Endoskop besteht demgegenüber im zusammengesetzten Zustand aus einem Beobachtungsteil und einem Beleuchtungsteil, die voneinander trennbar sind. Im Falle eines Defekts ist ein unmittelbarer Austausch der Beobachtungsgruppe auch im Operationssaal möglich, da die zylindrische Beobachtungsbaugruppe sich leicht aus der hohlzylindrischen Beleuchtungsbaugruppe herausziehen läßt.

Nachteilig bei diesem bekannten Endoskop ist aber dennoch, daß die kombinierte Spül- und Absaugvorrichtung nur nach der Entfernung des Beobachtungsteils aus dem Innenrohr der hohlzylindrischen Beleuchtungsbaugruppe zum Einsatz kommen kann, da dieses Innenrohr erst bei entferntem Beobachtungsteil als Spülkanal angewendet werden kann. Dadurch kann der Erfolg eines Spül- bzw. Absaugvorgangs, bei dem beispielsweise ein Gas zur Aufblähung des Gelenkinnenraumes injiziert wird, nicht visuell überwacht werden.

Aus der DE-A-37 27 190 ist ein an seinem distalen Ende geschlossenes Führungsrohr bekannt, in welches ein Endoskop einführbar ist. Bei einer ersten Ausführungsform ist das Führungsrohr als Lichtleiter ausgebildet, so daß das in ihn einzuführende Endoskop keine Beleuchtungsbaugruppe enthalten muß. Bei einer weiteren Ausführungsform weist das Führungsrohr keine Beleuchtungsbaugruppe auf, wohingegen das in ihn einführbare Endoskop diese aufweist. Hierbei wird der Hohlraum zwischen dem Endoskop und dem Führungsrohr mit einer Salzlösung durchströmt, um zu verhindern, daß sich Wassertröpfchen an der Innenfläche des Führungsrohrs bilden, die das vom Endoskop ausgegebene Licht streuen könnten und somit die erzielbare Sichtqualität verschlechtern. Diese Spülvorrichtung ist aber nicht mit einer Spül- und Absaugvorrichtung für vom Körperinnenraum abzusaugende oder in den Körperinnenraum einzuleitende Flüssigkeiten oder Gase gleichzusetzen, da das durch das Führungsrohr strömende Medium nicht in den Körperinnenraum gelangen kann und lediglich zur Verbesserung der Sichtqualität dient.

Der Erfindung liegt die Aufgabe zugrunde, ein Endoskop der eingangs genannten Gattung derart zu verbessern, daß die visuelle Überwachung eines Spül- bzw. Absaugvorgangs, bei dem Flüssigkeiten oder Gase vom zu untersuchenden Körperinnenraum abgesaugt bzw. in ihn eingeleitet werden bei gleichzeitig geringem Endoskopquerschnitt und guter Beleuchtung durchführbar ist.

Diese Aufgabe wird mit den kennzeichnenden Merkmalen des Anspruchs 1 gelöst.

Die Erfindung beruht auf der Erkenntnis, daß zwischen dem optiktragenden Schaft der Beobachtungsbaugruppe und dem doppelwandigen, die Lichtleiter aufnehmenden, Hohlzylinder der Beleuchtungsbaugruppe ein Hohlraum zum Durchtritt der Spül- oder Absaugflüssigkeiten bzw. -gase vorgesehen ist. Im Gegensatz zu herkömmlichen Endoskopen bzw. Arthroskopen bildet dabei nicht das Absaugrohr die äußere Begrenzung des rohrförmigen Schaftes des Arthroskops, sondern der die Lichtleitfasern aufnehmende doppelwandige Hohlzylinder. Folglich steht zur Aufnahme der Lichtleitfasern ein ringförmiger Bereich mit größeren Durchmessern zur Verfügung und es kann eine größere Zahl von Lichtleitfasern hindurchgeführt und konzentrisch um den optiktragenden Schaft angeordnet werden. Damit ergibt sich eine wesentliche Steigerung der Beleuchtungsintensität.

Dadurch, daß Absaug- und Spülkanal nicht separat auf das Endoskop aufgeschoben werden müssen, sondern in dessen Schaft zwischen Beobachtungs- und Beleuchtungseinheit "integriert" sind, ergibt sich eine Ersparnis bezüglich des Wandungsquerschnitts, welche nicht nur eine Vergrößerung des Beleuchtungs- sondern auch des Beobachtungsquerschnitts ermöglicht. Damit kann beispielsweise der Durchmesser des Beobachtungskanals (Linsendurchesser) von bisher 2,7 auf 3,0 mm gesteigert werden. Da das erzielbare optische Auflösungsvermögen quadratisch mit der Apertur zunimmt, die ihrerseits linear vom Linsendurchmesser abhängt, ist die auf diese Weise erzielbare Verbesserung beträchtlich.

Am Ausgang des distalen, in den Gelenkinnenraum einzuführenden rohrförmigen Teiles des Arthroskops befindet sich ein Anschlußstutzen zur Lichtzuführung für die Lichtleitfaserbeleuchtungseinrichtung. Direkt darüber oder durch ein Distanzrohr getrennt, lassen sich wahlweise spezielle ventilartige Schleusen für den Absaug- und/oder Spülmechanismus einrasten, einschrauben oder in anderer Weise befestigen. Ein sich anschließendes Führungsrohr gestattet einerseits ein exakt zentrisches Einsetzen der Beobachtungsbaugruppe des Endoskops und andererseits einen wirksamen Schutz des Optikschaftes vor mechanischer Überbelastung bzw. vor Beschädigung durch Instrumente. Die Beobachtungsbaugruppe ist okularseitig mit dem Führungsrohr verriegelbar.

Im eingeschobenen Zustand weisen der optiktragende Schaft der Beobachtungsbaugruppe und die doppelwandig hohlzylindrische Umhüllung für die Lichtleitfasern der Beleuchtungsbaugruppe annähernd die gleiche distale Länge auf. Ein Beschnitt des optischen Sehfeldes wird dadurch vermieden.

Um Gewebebeschädigungen während des Einführens des Endoskops zu vermeiden, ist dessen distales Ende unter Einbeziehung einer objektseitig konvexen Form der Frontlinse vorzugsweise abgerundet ausgebildet.

Durch Lösen einer Verriegelung zwischen der Beobachtungsbaugruppe und dem Führungsrohr und teilweisem Herausziehen der Beobachtungsbaugruppe, ergibt sich eine einfache Möglichkeit, das Objektiv der Beobachtungsbaugruppe freizuspülen und von eventuellen Verunreinigungen zu befreien. Diese an sich bekannte Methode wirkt bei der erfindungsgemäßen Anordnung der Baugruppen jedoch wesentlich effektiver als bei herkömmlichen Anordnungen, da die Spül- bzw. Saugflüssigkeit das Objektiv unmittelbar umspült. Außerdem wird die innere Wandung des Saug-/Spülkanals direkt von dem optiktragenden Schaft gebildet.

Der Blickwinkel, z.B. 90° (Weitwinkeloptik) und die Blickrichtung, z.B. 0°, 30° oder 70° zur Längsachse des Athroskops sind durch die schnelle und bequeme Austauschbarkeit der Beobachtungsbaugruppe auch während des Operationsvorganges frei wählbar. Durch Drehung der Beobachtungsbaugruppe innerhalb des Führungsrohres läßt sich bei Blickrichtungen ungleich 0° ein Überblick über Nachbarzonen des Operationsgebietes gewinnen. Die gewählte Beleuchtungsbaugruppe ist entsprechend dem gewünschten Ausleuchtungsbereich des Objektbereiches auszuwählen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein vorteilhaftes Ausführungsbeispiel der Beobachtungsbaugruppe als Teils des erfindungsgemäßen Endoskops,
Figur 2 das erfindungsgemäße Endoskop in der Ausführung als Athroskop in montiertem Zustand - teilweise im Schnitt dargestellt -
Figur 3 einen Schnitt in Richtung A-A gemäß Figur 2 in vergrößerter Wiedergabe,
Figur 4 einen Schnitt in Richtung B-B gemäß Figur 2 in vergrößerter Wiedergabe,
Figur 5 einen Schnitt in Richtung C-C gemäß Figur 2 in vergrößerter Wiedergabe sowie
Figur 6 eine Variante des zuvor dargestellten Endoskops in perspektivischer Darstellung.

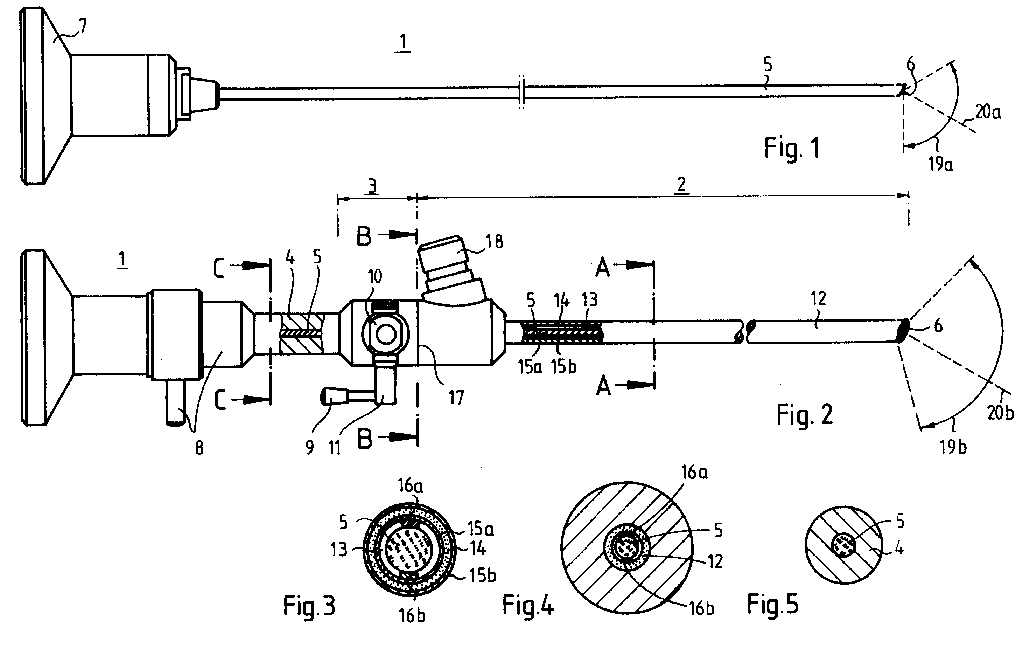

Die nachfolgende Beschreibung erfolgt unter gleichzeitiger Bezugnahme auf die Figuren 1 und 2, wobei Figur 2 das vollständige Endoskop und Figur 1 die aus dem Beleuchtungsteil herausgezogene Beobachtungsbaugruppe separat zeigt.

Das erfindungsgemäße Endoskop in der Ausführung als Arthroskop besteht aus einer Beobachtungsbaugruppe 1, einer Beleuchtungsbaugruppe 2, einer den Anschluß oder die Anschlüsse für die Saug- oder/und Spülvorrichtung enthaltenden Baugruppe 3 und einem Führungsrohr 4. Alle Hauptbestandteile 1 bis 4 des Arthroskops können für die verschiedensten Anwendungsfälle sehr unterschiedliche Bauformen aufweisen. Über die vorgesehenen, weiter unten beschriebenen, entsprechenden Kupplungsstellen lassen sich viele Kombinationen realisieren.

Die Beobachtungsbaugruppe 1 setzt sich aus einem optiktragenden Schaft 5, einem Objektiv 6 am distalen Ende des Schaftes 5, einem Okular 7 und Kupplungselementen 8 am okularseitigen Ende des Schaftes 5 zusammen. Die Kupplungselemente 8 ermöglichen eine Klemmverbindung der Beobachtungsbaugruppe 1 und des Fuhrungsrohres 4. Das Führungsrohr 4 dient einerseits der Zentrierung des eingeschobenen stabförmigen Schaftes 5 und andererseits dem mechanischen Schutz des Schaftes 5. An seinem anderen Ende ist das Führungsrohr 4 mit der den Zu- oder/und Abfluß von Saug oder/und Spülsubstanz regelnden Baugruppe 3 der Saugoder/und Spüleinrichtung verbunden. Die Baugruppe 3 ermöglicht über einen mit einem Hebel 9 zu betätigenden ventilartigen Verschluß 10 den Abfluß oder Zufluß von Flüssigkeiten oder Gasen über mindestens einen radial zur Längsachse des Arthroskops abstehenden Hohlstutzen 11.

An den Hohlstutzen 11 ist ein Schlauch anschließbar, der dem Zu- oder Abfluß der Flüssigkeiten bzw. Gase dient. Die Leitung der Saug- oder Spülsubstanz innerhalb der in den Gelenkinnenraum einzuführenden Kanüle 12 erfolgt in einem im Querschnitt ringförmigen Hohlrohr 13. Die innere Wandung des Hohlrohres 13 wird von dem zylindrischen Schaft 5 der Beobachtungsbaugruppe 1 gebildet und die äußere Wandung ist gleichzeitig innere Begrenzung eines Lichtleitfasern 14 führenden doppelwandigen Hohlzylinders, welcher aus der inneren Wandung 15a und der äußeren Wandung 15b besteht, wobei die äußere Wandung 15b gleichzeitig die äußere Begrenzung der in den Gelenkinnenraum einzuführenden Kanüle 12 bildet. Die innere Wandung 15a des Beleuchtungskanals kann über ihre gesamte Länge mit zwei vorzugsweise einander gegenüberliegenden Längsstegen 16a und 16b versehen sein (Figur 3). Die Längsstege 16a und 16b dienen einerseits als Abstandshalter zwischen dem optiktragenden Schaft 5 und der inneren Wandung 15a des die Lichtleitfasern 14 führenden doppelwandigen Hohlzylinders und teilen andererseits das Hohlrohr 13 in zwei separate Kammern mit vorzugsweise gleichgroßem ringsegmentförmigem Querschnitt, so daß bei Vorhandensein mehrerer Flüssigkeits- oder Gasströme deren vollständige Trennung möglich ist.

Der doppelwandige Hohlzylinder 15a/15b ist an einer Kupplungsstelle 17 mit der dem distalen Ende des Arthroskops zugewandten Seite der den Verschluß 10 der Saug-/Spülvorrichtung enthaltenden Baugruppe 3 verbunden.

Der Querschnitt an der Kupplungsstelle 17 ist in Figur 4 dargestellt. Die den ventilartigen Verschluß 10 zur Einleitung oder zum Absaugen von Substanzen enthaltende Baugruppe 3 der Saug- oderund Spülvorrichtung ist vom distalen Ende des Arthreoskops aus gesehen der einen Beleuchtungsstutzen 18 enthaltenden Baugruppe nachgeordnet. Der radial zur Längsachse des Arthroskops abstehende Beleuchtungsstutzen 18 dient dem Anschluß von selbsttragenden oder nicht-selbsttragenden (flexiblen) Lichtleitkabeln.

Die Kupplungsstelle 17 ermöglicht neben dem Anschluß verschiedener ventilartige Verschlüsse enthaltender Baugruppen 3 auch den direkten Anschluß eines Führungsrohres 4 an die den Beleuchtungsstutzen 18 enthaltende Baugruppe. Das den Saug- oder/und Spülkanal bildende Hohlrohr 13 bleibt in einem solchen Anwenendungsfall ungenutzt. Figur 5 zeigt einen Querschnitt durch ein Führungsrohr.

Die Länge des Führungsrohres ist so bemessen, daß das distale Ende der Beobachtungsbaugruppe 1 mit dem distalen Ende der Beleuchtungsbaugruppe 2 bei bis zum Anschlag eingeschobener Beobachtungsbaugruppe 1 annähernd übereinstimmt. Damit ist gewährleistet, daß der ausgeleuchtete Objektraum mit dem zu beobachtenden Objektraum nahezu identisch ist, vorausgesetzt, die Beobachtungsbaugruppe 1 ist mit der Beleuchtungsbaugruppe 2 so gepaart, daß der Blickwinkel 19a und die Blickrichtung 20a der Beobachtungtsbaugruppe 1 dem Beleuchtungswinkel 19b und der Beleuchtungsrichtung 20b entspricht. Um die notwendige Übereinstimmung insbesondere in Bezug auf Blickrichtung 20a und Beleuchtungsrichtung 20b schnell und bequem einstellen zu können, ist die Beobachtungsbaugruppe 1 mittels der Kupplungselemente 8 in der eingestellten Winkelposition relativ zur Beleuchtungsbaugruppe 2 klemmbar.

Zum reinigenden Abspülen des Objektivs 6 kann die Klemmung der Beobachtungsbaugruppe 1 mittels der Kupplungselemente 8 gelockert merden, so daß sich die Beobachtungsbaugruppe 1 etwas herausziehen läßt. Wird eine Beobachtungsbaugruppe 1 mit einer Blickrichtung verwendet, die von der Richtung der Längsachse des Arthroskops abweicht, ergibt sich nach der Lockerung der Kupplungselemente 8 durch die freie Drehbarkeit der Beobachtungsbaugruppe 1 innerhalb des Führungsrohres 4 die Möglichkeit eines "Rundumblickes".

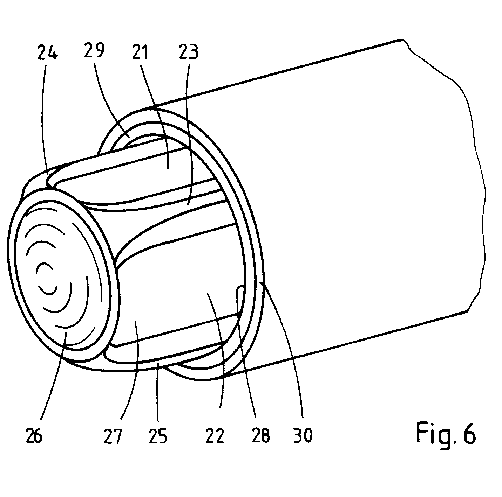

Figur 6 zeigt das distale Ende eines Endoskops als Variante zu der in Figur 3 dargestellten Ausführung in perspektivischer Darstellung. Dabei sind zwei Flüssigkeits- bzw. Gaskanäle 21 und 22 vorgesehen, die durch Längsstege 23 bis 25 voneinander bzw. von einem weiteren in der Darstellung nicht sichtbaren Kanal getrennt sind.

Die Längsstege 23 und 24 verjüngen sich in radialer Richtung und verbreiten sich zum distalen Ende hin. Im Längsschnitt sind die Stege derart verrundet, daß am distalen Ende ihre Längenabmessung in radialer Richtung abnimmt.

Die dargestellte Ausführungsform weist damit eine auch in ihrer Außenkontur zum distalen Ende hin vollständig verrundete Spitze auf, wodurch ein leichtes, gewebeschonendes und verhakungsfreies Einführen der Endoskopoptik möglich ist.

Die Krümmung einer objektseitig konvex gewölbten Objektivlinse 26 geht dabei stetig in die stirnseitige Krümmung eines optiktragenden Schaftes 27, einer inneren Wandung 28, einer Lichtaustrittsfläche von Lichtleitfasern 29 und einer äußeren Wandung 30 über, vorausgesetzt, die Beobachtungsbaugruppe ist bis zum Anschlag in die die innere Wandung 28, die Lichtleitfasern 29 und die äußere Wandung 30 aufweisende Beleuchtungsbaugruppe eingeschoben. Durch die Verbreiterung der Stege im Fußbereich kann hier die Verrundung einem eventuellen Verkaken beim Einführen besonders günstig entgegenwirken. Die Stege sind in radialer Richtung in ihren Abmessungen so begrenzt, daß sie das Verschieben des optiktragenden Schaftes gegenüber dem Beleuchtungsteil nicht behindern.

Als besonders vorteilhaft erweist sich die problemlose und schnelle Auswechselbarkeit der Beobachtungsbaugruppe 1, die auch während des Operationsvorganges möglich ist. Beispielsweise können nacheinander Optiken verschiedener Aperturen oder verschiedener Blickrichtungen eingesetzt werden.

Da erfindungsgemäß die äußere Wandung 15b des Beleuchtungskanals die äußere Begrenzung der Kanüle 12 bildet und nicht - wie bei herkömmlichen Arthroskopen üblich - der Saug- bzw. Spülkanal, lassen sich mehr Lichtleitfasern unterbringen, woraus eine erheblich gesteigerte Beleuchtungsintensität resultiert.

Die verringerten Wandstärken gestatten außerdem eine erhebliche Vergrößerung des Durchmessers des optiktragenden Schaftes 5 und damit des freien Durchmessers des Objektivs 6. Die auf diese Weise erzielbare Aperturerhöhung bewirkt eine starke Verbesserung des Auflösungsvermögens.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Endoskop, insbesondere Arthroskop, zur Untersuchung von Körperinnenräumen, bestehend aus einem optiktragenden Schaft (5), um den Schaft (5) angeordneten Lichtleitern (14) und einer konzentrisch um den Schaft (5) angeordneten schleusenbetriebenen Absaug- und/oder Spülvorrichtung für vom Körperinnenraum abzusaugende oder in den Körperinnenraum einzuleitende Flüssigkeiten oder Gase, wobei der optiktragende Schaft (5) Teil einer Beobachtungsbaugruppe (1) ist und die Lichtleiter (14) Teil einer Beleuchtungsbaugruppe (2) sind, wobei die Beleuchtungsbaugruppe (2) eine doppelwandige aus zwei Hohlzylindern bestehende Umhüllung aufweist und die Lichtleiter (14) zwischen den durch die Hohlzylinder gebildeten Wandungen (15a und 15b) geführt sind und die Beobachtungsbaugruppe (1) in die Beleuchtungsbaugruppe (2) einschiebbar ist,
**dadurch gekennzeichnet,**
daß die Lichtleiter (14) auf dessen gesamter Länge um den optiktragenden Schaft (5) angeordnet sind und daß ein Hohlraum (13) zwischen dem optiktragenden Schaft (5) und der inneren Wandung (15a) der Beleuchtungsbaugruppe (2) einen Absaug- und/oder Spülkanal für die Absaug- und/oder Spülvorrichtung bildet.

2. Endoskop nach Anspruch 1, **dadurch gekennzeichnet,** daß zwischen Beobachtungs- und Beleuchtungsbaugruppe (1 und 2) mindestens ein längsgerichteter Führungssteg (16a oder 16b) als Abstandhalter vorgesehen ist, wobei bei zwei Führungsstegen (16a und 16b) diese insbesondere eine Trennung zwischen Absaug- und Spülkanal bilden.

3. Endoskop nach einem der vorangehenden Ansprüche, **dadurch gekennzeichnet,** daß der Anschluß (11) oder die Anschlüsse für die Absaug- und/oder Spülvorrichtung, vom distalen Ende her gesehen, hinter dem Anschluß (18) für die Lichtquelle vorgesehen ist bzw. sind.

4. Endoskop nach Anspruch 3, **dadurch gekennzeichnet,** daß die Absaug- und/oder Spülvorrichtung eine abtrennbare Baugruppe (3) aufweist, welche koaxial ausgerichtet an das den Beleuchtungsstutzen (18) aufweisende Ende der Beleuchtungsbaugruppe (2) anfügbar ist, wobei die dem distalen Ende zugewandte Seite einen Öffnungsbereich aufweist, der dem Absaug- bzw. Spülkanal angepaßt ist und im übrigen einen nach außen hin dichtenden Abschluß aufweist, während die gegenüberliegende Stirnfläche einen Durchlaß lediglich für den Schaft (5) der Beobachtungsbaugruppe (1) aufweist.

5. Endoskop nach einem der vorangehenden Ansprüche, **dadurch gekennzeichnet,** daß die distalen Enden der Beobachtungsbaugruppe (1) und der Beleuchtungsbaugruppe (2) im eingeschobenen Zustand in ihrer Länge übereinstimmen bzw. bis zu wenigen Millimetern voneinander abweichen.

6. Endoskop nach einem der vorangehenden Ansprüche, **dadurch gekennzeichnet,** daß das distale Ende der Beobachtungsbaugruppe (1) durch Stege (23, 24) innerhalb der Beleuchtungsbaugruppe (2) zentriert ist, die an ihrem objektseitigen Ende mit in radialer Richtung zunehmender Verkürzung verrundet sind, wobei insbesondere auch die Stegbreite in radialer Richtung abnimmt.

7. Endoskop nach einem der vorangehenden Ansprüche, **dadurch gekennzeichnet,** daß die Beleuchtungsrichtung (20b) und der Beleuchtungswinkel (19b) der Beleuchtungsbaugruppe (2) auf die Blickrichtung (20a) und den Blickwinkel (19a) der Beobachtungsbaugruppe (1) abgestimmt sind.

8. Endoskop nach einem der vorangehenden Ansprüche, **dadurch gekennzeichnet,** daß die Beobachtungs- und die Beleuchtungsbaugruppe (1 und 2) relativ zueinander verdreh- und/oder arretierbar sind.

9. Endoskop nach einem der vorangehenden Ansprüche, **dadurch gekennzeichnet,** daß die Lichtleiter (14) als Glasfaserleiter ausgebildet sind.

## Claims

1. Endoscope, particularly an arthroscope for investigating body cavities, comprising a shaft (5) which carries an optical member, light carriers (14) arranged around the shaft (5) and a valve-operated suction and/or rinsing device, arranged concentrically about the shaft (5), for liquids or gases which are to be sucked out of the body cavity or introduced into the body cavity, the shaft (5) carrying the optical member being part of an observation assembly (1) and the light conductors (14) being part of a lighting assembly (2), the lighting assembly (2) having a double-walled shell comprising two hollow cylinders and the light conductors (14) being guided between the walls (15a and 15b) formed by the hollow cylinders, the observation assembly (1) being insertable into the lighting assembly (2), characterised in that the light conductors (14) are arranged around the shaft (5) carrying the optical member and are provided along the entire length of said shaft and a cavity (13) between the shaft (5) which carries the optical member and the inner wall (15a) of the lighting assembly (2) forms a suction and/or rinsing channel for the suction and/or rinsing device.

2. Endoscope according to claim 1, characterised in that, between the observation and lighting assemblies (1 and 2), there is at least one longitudinally directed guide bar (16a or 16b) to act as a spacer, whilst if two guide bars (16a and 16b) are provided they form, in particular, a partition between the suction and rinsing channels.

3. Endoscope according to one of the preceding claims, characterised in that the connection (11) or connections for the suction and/or rinsing device, viewed from the distal end, is or are provided behind the connection (18) for the light source.

4. Endoscope according to claim 3, characterised in that the suction and/or rinsing device has a removable assembly (3) which is adapted to be fitted on, in coaxial alignment, to the end of the lighting assembly (2) which comprises the lighting connector (18), the end facing the distal end having an opening area which is adapted to fit the suction or rinsing channel and furthermore having a closure which seals outwardly, whilst the end face opposite has an opening exclusively for the shaft (5) of the observation assembly (1).

5. Endoscope according to one of the preceding claims, characterised in that the distal ends of the observation assembly (1) and lighting assembly (2), in the pushed-in state, correspond in length or differ in length by up to a few millimetres.

6. Endoscope according to one of the preceding claims, characterised in that the distal end of the observation assembly (1) is centred by bars (23,24) inside the lighting assembly (2), these bars being rounded off, at their end closest to the object, becoming progressively shorter in the radial direction, whilst the width of the bars, in particular, also decreases in the radial direction.

7. Endoscope according to one of the preceding claims, characterised in that the direction (20b) and angle (19b) of illumination of the lighting assembly (2) are adapted to the direction of viewing (20a) and the viewing angle (19a) of the observation assembly (1).

8. Endoscope according to one of the preceding claims, characterised in that the observation and lighting assemblies (1 and 2) are rotatable and/or lockable in position relative to one another.

9. Endoscope according to one of the preceding claims, characterised in that the light conductors (14) take the form of glass fibre conductors.

## Revendications

1. Endoscope, en particulier arthroscope pour examiner des cavités du corps, composé d'une tige (5) portant l'optique, des fibres optiques (14) agencées autour de la tige (5) et un dispositif d'aspiration et/ou de rinçage agencé concentriquement autour de la tige (5), fonctionnant par éclusage, pour aspirer des cavités du corps ou pour y introduire des liquides ou des gaz, la tige (5) portant l'optique faisant partie d'un bloc d'observation (1) et les fibres optiques (14) faisant partie d'un bloc d'éclairage (2), le bloc d'éclairage (2) présentant une enveloppe à double paroi constituée de deux cylindres creux et les fibres optiques (14) étant guidées entre les parois (15a et 15b) formées par les cylindres creux, le bloc d'observation (1) pouvant être introduit dans le bloc d'éclairage (2),
caractérisé en ce que
les fibres optiques (14) sont disposées autour de la tige (5) portant l'optique, sur toute sa longueur, et en ce qu'une cavité (13) entre la tige (5) portant l'optique et la paroi intérieure (15a) du bloc d'éclairage (2) forme un canal d'aspiration et/ou de rinçage pour le dispositif d'aspiration et/ou de rinçage.

2. Endoscope selon la revendication 1, caractérisé en ce qu'entre les blocs d'observation et d'éclairage (1 et 2), il est prévu au moins une nervure de guidage longitudinale (16a ou 16b) servant de pièce d'écartement, et dans le cas de deux nervures de guidage (16a et 16b), celles-ci forment en particulier une séparation entre le canal d'aspiration et le canal de rinçage.

3. Endoscope selon l'une quelconque des revendications précédentes, caractérisé en ce que le raccordement (11) ou les raccordements pour le dispositif d'aspiration et/ou de rinçage est ou sont prévus, vus de l'extrémité distale, derrière le manchon de raccordement (18) pour la source de lumière.

4. Endoscope selon la revendication 3, caractérisé en ce que le dispositif d'aspiration et/ou de rinçage présente un bloc (3) amovible qui est peut être ajouté en alignement coaxial à l'extrémité du bloc d'éclairage (2) présentant le manchon d'éclairage (18), le côté orienté vers l'extrémité distale présentant une région d'ouverture qui est adaptée au canal d'aspiration et de rinçage et en outre une fermeture étanche vers l'extérieur, tandis que la face opposée présente un passage uniquement pour la tige (5) du bloc d'observation (1)

5. Endoscope selon l'une quelconque des revendications précédentes, caractérisé en ce que les extrémités distales du bloc d'observation (1) et du bloc d'éclairage (2) coïncident du point de vue de leur longueur ou divergent de quelques millimètres, lorsqu'ils sont emboîtés l'une dans l'autre.

6. Endoscope selon l'une quelconque des revendications précédentes, caractérisé en ce que l'extrémité distale du bloc d'observation (1) est centrée par des nervures (23, 24) à l'intérieur du bloc d'éclairage (2), lesquelles sont arrondies à leur extrémité tournée vers l'objet en se rétrécissant en direction radiale, la largeur des nervures diminuant en particulier aussi en direction radiale.

7. Endoscope selon l'une quelconque des revendications précédentes, caractérisé en ce que la direction d'éclairage (20b) et l'angle d'éclairage (19b) du bloc d'éclairage (2) sont accordés sur la direction de visée (20a) et l'angle de visée (19a) du bloc d'observation (1).

8. Endoscope selon l'une quelconque des revendications précédentes, caractérisé en ce que les blocs d'observation et d'éclairage (1 et 2) peuvent être tournés ou bloqués relativement l'un à l'autre.

9. Endoscope selon l'une quelconque des revendications précédentes, caractérisé en ce que les fibres optiques (14) sont réalisées sous la forme de fibres de verre.
